(19) Europäisches Patentamt
European Patent Office
Office européen des brevets

(11) Veröffentlichungsnummer: **0 194 571**
**B1**

(12) **EUROPÄISCHE PATENTSCHRIFT**

(45) Veröffentlichungstag der Patentschrift: **14.11.90**

(21) Anmeldenummer: **86102901.5**

(22) Anmeldetag: **05.03.86**

(51) Int. Cl.$^5$: **A 61 K 31/33**, A 61 K 31/415, A 61 K 31/53, C 07 D 235/28, C 07 D 249/08, C 07 D 253/04, C 07 D 277/32

(54) **Heterocyclische Disulfide, Verfahren zu ihrer Herstellung und pharmazeutische Mittel, die diese Verbindungen enthalten.**

(30) Priorität: **12.03.85 DE 3508666**

(43) Veröffentlichungstag der Anmeldung: **17.09.86 Patentblatt 86/38**

(45) Bekanntmachung des Hinweises auf die Patenterteilung: **14.11.90 Patentblatt 90/46**

(84) Benannte Vertragsstaaten: **AT BE CH DE FR GB IT LI LU NL SE**

(56) Entgegenhaltungen:
**EP-A-0 061 425**
**EP-A-0 077 630**
**DD-A- 153 837**
**DE-A-2 120 995**
**DE-A-2 145 296**
**DE-A-3 118 128**
**FR-A-2 403 798**
**FR-M- 5 680**
**GB-A-1 327 466**
**US-A-4 152 439**
**US-A-4 378 364**

**CHEMICAL ABSTRACTS, Bd. 49, 10. Januar 1955, Columbus, Ohio, US; J. RENAULT "The sulfurization of 2-pyridones and 2-piperidones", Spalte 321, Zusammenfassung-Nr. 321a**

(73) Patentinhaber: **HOECHST AKTIENGESELLSCHAFT**
**Postfach 80 03 20**
**D-6230 Frankfurt am Main 80 (DE)**

(72) Erfinder: **Fleischmann, Klaus, Dr.**
**In den Weingärten 24**
**D-6236 Eschborn (DE)**
Erfinder: **Dürckheimer, Walter, Dr.**
**Im Lerchenfeld 45**
**D-6234 Hattersheim am Main (DE)**
Erfinder: **Blumbach, Jürgen, Dr.**
**Manderscheider Strasse 13b**
**D-6000 Frankfurt am Main 71 (DE)**
Erfinder: **Limbert, Michael, Dr.**
**Am Alten Birnbaum 21**
**D-6238 Hofheim am Taunus (DE)**
Erfinder: **Schorlemmer Hans-Ulrich, Dr.**
**Am Kirschenwald 2**
**D-3550 Marburg 21 (DE)**
Erfinder: **Dickneite, Gerhard, Dr.**
**Zum Neuen Hieb 31**
**D-3550 Marburg (DE)**
Erfinder: **Sedlacek, Hans-Harald, Dr.**
**Sonnenhand 3**
**D-3550 Marburg (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 194 571 B1

## Beschreibung

Es ist bekannt, daß die Abwehrmechanismen des lebenden Organismus, die kurz als humorale Immunität und zelluläre Immunität bezeichnet werden, zusammenwirken, um Fremdkörper, die pathogenetische Veränderungen hervorrufen und schädlich sein können, zu neutralisieren und zu eliminieren, vornehmlich Mikroorganismen oder neoplastische Zellen.

Immunlogische Untersuchungen ergaben, daß Zusammenhänge zwischen der durch innere oder durch äußere Faktoren provozierten Abnahme der immunlogischen Aktivität und der Zunahme der Infektions- oder Tumorkrankheiten bestehen. Daneben entstehen andere Krankeiten durch Veränderungen der Funktionen des Immunsystems. Hierzu gehören besipielsweise Autoimmunkrankheiten oder durch Immunkomplexe hervorgerufene Erkrankungen. Man sucht deshalb seit langem nach Immunstimulantien, d.h. nach Substanzen, die imstande sind, die immunologische Aktivtät des Empfängers zu verändern, vorzugsweise zu erhöhen und die aufgrund ihrer hohen Wirksamkeit und guten Verträglichkeit einen breiten Einsatz zur Unterstützung der Abwehrkräfte des Körpers erlauben. Beispiele, die hinsichtlich der Stimulation der Immunität geprüft werden, sind BCG und C. parvum, ferner die Extrakte des M. tuberculosis und der Brucellen.

Diese Substanzen erzeugen jedoch in den Konzentrationen, wie sie zur Anwendung kommen, deutliche Nebenwirkungen, wie z.B. in verschiedenem Ausmaß lokale Granulome. Die Unkenntnis der genauen Natur der Substanzen erschwert eine systematische Untersuchung mit guter Reproduzierbarkeit der klinischen Ergebnisse. Erwünscht sind somit in diesem Zusammenhang neue Immunstimulantien, die chemisch definierte Substanzen darstellen und geringe Toxizität besitzen, wie z.B. Bestatin, das zur Zeit ein intensiv untersuchtes niedermolekulares Immunstimulanz ist und allgemein eine wissenschaftliche Referenzsubstanz darstellt.

In US—A—4,378,364 werden bestimmte Bis(hetero)disulfide beschrieben, deren heterocyclischer Ring auch durch Carboxylgruppen substituiert sein kann. Die Verabreichung dieser Verbindungen an Krebspatienten nach der Operation soll zu einer Verbesserung des Wohlbefindens, der Aktivität, des Appetits, der Gewichtszunahme und zu einer Schmerzminderung führen. Dies wird in verschiedenen Beispielen mit der 6,6'-Dithiodinicotinsäure (CPDS) gezeigt.

Überraschenderweise wurde nun gefunden, daß die erfindungsgemäßen Verbindungen eine hohe immunstimulierende und immunrestaurative Wirkung besitzen, wie sie sich beispielsweise in der DTH-Reaktion auf Schafserythrozyten, in der Aktivierung von mononucleären Phagozyten und in einer ausgeprägten CSF-Aktivität ausdrückt. Diese immunstimulierenden Effekte können beispielswiese auch in einer Erhöhung der Widerstandskraft gegen Infektionen beobachtet werden. Darüberhinaus besitzen die erfindungsgemäßen Verbindungen überraschenderweise zytostatische Wirksamkeit, so beispielsweise gegen das B16-Melanom an der Maus.

Die Erfindung beschreibt somit eine Klasse von immunpharmakologisch und zytostatisch wirksamen Substanzen, die chemisch definiert sind, geringe Toxizität besitzen und als solche oder in Kombination mit anderen Wirkstoffen wertvolle Arzneimittel darstellen. Die erfindungsgemäßen Verbindungen weisen einen $LD_{50}$-Wert von mehr als 1000 mg/kg bei intravenöser Injektion bei Mäusen auf. Die wirksame immunmodulatorische und zytostatische Menge liegt bei Wirbeltieren, vorzugsweise warmblütigen Säugern, im Bereich von etwa 0,5 bis etwa 100 mg/kg Körpergewicht pro parenteraler oder oraler Gabe, ohne dabei toxische Nebenwirkungen zu zeigen und ist damit für die Behandlung von Krankheiten des Immunsystems sehr gut geeignet.

Die Erfindung betrifft als Disulfide der allgemeinen Formel I'

$$\text{Het'—S—S—Het'} \qquad (I')$$

in der Het' die Bedeutung besitzt von 1,3-Thiazolyl, das substituiert ist durch $C_1$—$C_6$-Alkyl; durch Carboxyl-$C_1$—$C_6$-alkyl; durch Carboxy oder durch $C_2$—$C_5$-Acylamino, wobei Acyl der Rest einer aliphatischen Dicarbonsäure ist; 1,2,4-Triazolyl, das substituiert ist durch Hydroxy und Phenyl; 1,3,4-Thiadiazolyl, das substituiert ist durch Carboxy-$C_1$—$C_4$-alkylthio, Dihydro-1,2,4-triazinyl, das substituiert ist durch $C_1$—$C_6$-Alkyl, Halogeno-$C_2$—$C_4$-alkenyl, Oxo oder Hydroxy oder Benzimidazolyl, das substituiert ist durch Carboxy, wobei Het' durch mindestens eine direkt oder an einen Substituenten gebundene Carboxygruppe oder eine direkt gebundene Hydroxygruppe, wobei diese beiden Gruppen auch in Form eines physiologisch verträglichen Salzes vorliegen können, substituiert ist.

Als $C_1$—$C_6$-Alkyl-Substituenten kommen in Betracht geradkettige oder verzweigte Alkylgruppen mit 1—6, vorzugsweise 1—3 C-Atomen, wie z.B. Methyl, Ethyl, n- oder i-Propyl, n- oder tert.-Butyl, vorzugsweise Methyl, die gegebenenfalls wiederum substituiert sein können durch Carboxy.

Für eine $C_2$—$C_5$-Acylaminogruppe, in der Acyl für den Rest einer aliphatischen Dicarbonsäure steht, seien beispielsweise genannt 3-Carboxy-propionyl und 4-Carboxy-butyryl.

Als durch Carboxy substituierte $C_1$—$C_4$-Alkylthioreste seien erwähnt z.B. Methylthio und Ethylthio, als Halogeno-$C_2$—$C_4$-alkenylreste z.B. Vinyl oder Allyl, die beispielsweise durch Chlor oder Brom substituiert sind.

Der Heterocyclus Het' kann ein- oder mehrfach, beispielsweise ein- bis dreifach substituiert sein. Bevorzugt sind jedoch solche Verbindungen, die im heterocyclischen oder im ankondensierten

carbocyclischen Ring einen oder 2 Substituenten tragen. Von diesen Substituenten muß mindestens einer eine saure Funktion besitzen. So kann ein Substituent entweder eine Carboxygruppe tragen, wie z.B. Carboxyalkyl oder Carboxyalkylthio, oder er kann als Carboxy- oder Hydroxygruppe auch direkt an den Heterocyclus Het' gebunden sein.

Erfindungsgemäß besonders bevorzugt sind Verbindungen, in denen Het' für einen Thiazolrest steht, der mindestens durch eine Carboxyl- oder eine Carboxymethylgruppe substituiert ist.

Da die Verbindungen der allgemeinen Formel I' saure Funktionen, wie insbesondere eine Carboxygruppe, tragen, können sie auch in Form ihrer physiologisch verträglichen Salze vorliegen, beispielswiese als Alkali- und Erdalkalisalze, wie vorzugsweise die Na, K, Ca, Mg-Salze oder beispielsweise Ammoniumsalze oder substituierte Ammoniumsalze, wie beispielsweise $NH_4^+$, Ethanolammonium, Diethanolammonium, Trialkylammonium, wie z.B. Triethylammonium, Tetraalkylammonium, Salze mit basischen Aminosäuren, wie z.B. Lysin, Arginin.

Gegenstand der Erfindung ist weiterhin ein Verfahren zur Herstellung der erfindungsgemäßen Verbindungen der allgemeinen Formel I'.

Das erfindungsgemäße Verfahren ist dadurch gekennzeichnet, daß man eine Verbindung der allgemeinen Formel II

$$\text{Het'—SH} \qquad (II)$$

in der Het' die oben angeführte Bedeutung hat, durch Umsetzung mit einem Oxidationsmittel in der erfindungsgemäßen Verbindungen überführt.

Als Oxidationsmittel seien beispielsweise genannt Sauerstoff, Wasserstoffperoxid, u.U. unter Zusatz von Eisensalzen, wie beispielsweise Mohr'schem Salz oder unter Zusatz von Basen, wie beispielsweise Natriumhydroxid, Kaliumhydroxid, Natriumhydrogencarbonat oder Kaliumhydrogencarbonat, organische Persäuren, wie beispielsweise Peressigsäure, Perbenzoesäure oder m-Chlorperbenzoesäure, elementares Jod oder Brom, u.U. unter Zusatz von Basen, wie beispielsweise Natriumhydroxid oder Kaliumhydroxid, $FeCl_3$ oder $K_3(Fe(CN)_6)$.

Als Oxidationsmittel seien als bevorzugt genannt Sauerstoff, Wasserstoffperoxid, organische Persäuren, wie beispielsweise Peressigsäure, Perbenzoesäure und m-Chlorperbenzoesäure oder elementaires Jod. Die Oxidation wird bevorzugt in Wasser oder einem organischen Lösungsmittel durchgeführt, wie beispielsweise Methanol, Ethanol, iso-Propanol, Essigester und halogenierten Kohlenwasserstoffen, wie beispielsweise Dichlormethan und Chloroform. Es kann auch ein Gemisch dieser Lösungsmittel eingesetzt werden. Beim Arbeiten mit Sauerstoff, Wasserstoffperoxid und Persäuren ist die Verwendung von Lösungsmitteln, die bekanntermaßen explosive Peroxide bilden können, wie beispielsweise Ether, Tetrahydrofuran, Dioxan, Aceton, Methylethylketon, iso-Propanol usw., zu vermeiden.

Die erfindungsgemäßen Verbindungen können auch hergestellt werden, indem man eine Verbindung der allgemeinen Formel III

$$\text{Het'—X} \qquad (III)$$

in der Het' die oben angeführte Bedeutung hat und X für eine reaktive Abgangsgruppe, wie Halogen, bevorzugt Chlor oder Brom, wie $—OSO_2R$, wobei R die Bedeutung von bevorzugt Methyl, Trifluormethyl, Phenyl, Tolyl oder Naphthyl hat, wie $—OPO(OR'_2)$, wobei R' die Bedeutung von bevorzugt Phenyl hat, steht, mit $Me_2S_2$ umsetzt, wobei Me für ein Alkalimetall, vorzugsweise Natrium, steht.

Weiterhin können die erfindungsgemäßen Verbindungen hergestellt werden, indem man eine Verbindung der allgemeinen Formel IV

$$\text{Het'—S—SO}_2\text{Me} \qquad (IV)$$

in der Het' und Me die oben angeführten Bedeutungen haben mit Jod in wässrigem Medium umsetzt.

Die Umsetzung kann in einem Temperaturbereich zwischen etwa −40°C und dem Siedepunkt des Lösungsmittels bzw. Lösungsmittelgemisches, bevorzugt zwischen etwa −10°C und etwa +40°C durchgeführt werden.

Der Wirkstoff kann allein gegeben oder aber auch mit einem oder mehreren, vorzugsweise einem anderen Arzneimittel kombiniert werden, die Infektionen, die beispielsweise durch Bakterien, Pilze oder Viren hervorgerufen werden, und Tumorkrankheiten günstig beeinflussen. Die Wirkstoffe können erfindungsgemäß sowohl parenteral als auch oral verabreicht werden. Für die parenterale Verabreichung kommen Lösungen oder Suspensionen des Wirkstoffes in einem pharmazeutisch verträglichen Vektor in Betracht, vorzugsweise Pflanzenöl, wie z.B. Erdnußöl oder Sesamöl, sowie alkoholische Lösungen des Wirkstoffes, z.B. in Ethanol, Propandiol oder Glycerin oder in Gemischen der vorgenannten Lösungsmittel. Zur Herstellung wäßriger Lösungen wird der Wirkstoff vorzugsweise in Form wasserlöslicher, physiologisch verträglicher Salze eingesetzt. Die Zubereitungen können die üblichen Hilfs- und Trägerstoffe enthalten. Als solche kommen beispielsweise Füllstoffe, Emulgatoren, Gleit- und Pufferstoffe und geschmackskorrigierende Agenzien in Frage.

Die Erfindung betrifft daher ebenfalls pharmazeutische Mittel zur Immunstimulation, Immunrestauration und zytostatischen Behandlung, die eine Verbindung der allgemeinen Formel I' enthalten.

Im folgenden wird die Einwirkung der Verbindungen auf die Immunantwort der Maus und ihre immunstimulierenden Aktivitäten in verschiedenen in vivo-Standardmethoden beispielhaft erläutert. Die herangezogenen verschiedenen Testmethoden sind bekanntermaßen für die Beurteilung von Immunstimulantien und deren Wirkqualität besonders gut geeignet.

Experiment 1
Wirkung auf die zelluläre immunologische Reaktion vom verzögerten Typ gegen Schafserythrozyten (delayed type hypersensitivity, DTH)

Es wurden Gruppen von 5 weiblichen NMRI-Mäusen mit einem Gewicht von 18—20 g intravenös entweder $10^6$ oder $10^9$ rote Blutkörperchen vom Schaf pro Tier verabreicht. Schafserythrozyten gelten in der Immunologie als Standardprüfsubstanz (Antigen) zur Auslösung von zellulären und humoralen Immunreaktionen. Im besonderen gibt dieser Test Auskunft über die Funktionsfähigkeit der T-Zellabhängigen Komponente (T-Helferzellen) des Immunsystems. Die gemäß Ausführungsbeispiel 8 erhaltene Prüfsubstanz (Bis-(5-Carboxymethyl-4-methyl-thiazol-2-yl)-disulfid) wurde an den Tagen -3, -2, -1 und 0 in den Konzentrationen 20 mg/kg, 30 mg/kg und 40 mg/kg in physiologischer Kochsalzlösung intraperitoneal zweimal täglich appliziert. Nach 5 Tagen wurden allen Tieren jeweils $2\times10^8$ Schafserythrozyten in die Fußsohle injiziert und 24 Stunden später wurde die Schwellung des Fußes gemessen. Die Fußschwellung wird durch eine Hautreaktion vom verzögerten Typ (delayed type hypersensitivity, DTH) ausgelöst und ist, wie dem Fachmann bekannt, ein Maß für die zelluläre Immunantwort (Collins, F. M und Mackaness, G. B., J. Immunol. *101*, 830—845, 1968). Die in der *Tabelle* 1 zusammengesetellten Ergebnisse veranschaulichen, daß es durch die Verabfolgung der erfindungsgemäß erhaltenen Substanz beispielsweise nach Immunisierung mit $10^6$ Schafserythrozyten, zur Steigerung der zellulären Immunantwort kommt. Ein Maximum der Stimulation kann in diesem Experimentalansatz bei der Gabe von 30 mg/kg Prüfsubstanz beobachtet werden.

TABELLE 1
Immunisierung von Mäusen mit Schafserythrozyten-Wirkung auf die zelluläre Immunantwort (DTH-Reaktion)

| 2×/Tag i.p. Applikation an Tag -3, -2, -1, 0 von | % Fußschwellung bei $10^6$ Erythrozyten |
|---|---|
| PBS* | 24,3∓1,3 |
| 20 mg/kg | 28,3∓6,7 |
| Prüfsubstanz 30 mg/kg | 36,5∓6,5 |
| 40 mg/kg | 31,4∓8,7 |

*PBS=Phosphatgepufferte Kochsalzlösung (NaCl: 8000 mg/l, KCl: 200 mg/l, $Na_2HPO_4 \cdot 2H_2O$: 1440 mg/l, $KH_2PO_4$: 200 mg/l.

Experiment 2
Einfluß auf die Stimulation der unspezifischen Immunität-Aktivierung von mononukleären Phagozyten

Hier wurde der Einfluß der nach Ausführungsbeispiel 8 erhaltenen Prüfsubstanz auf die Stimulation von Peritonealmakrophagen bei 6—8 Wochen alten NMRI-Mäusen untersucht. Mäuseweibchen erhielten auf parenteralem oder oralem Wege die Prüfsubstanz in einer Dosis von 25 mg/kg, 50 mg/kg, 100 mg/kg und 200 mg/kg. Der Kontrollgruppe wurde gepufferte Kochsalzlösung verabreicht. Drei Tage nach den Injektionen wurden die Mäuse getötet, und es wurden die Peritonealmakrophagen der Tiere auf ihren Aktivierungszustand hin untersucht. Als Maß der Makrophagenaktivierung wurde zum einen die Sekretion der lyosomalen Enzyme (β-Galactosidase β-Glucuronidase, N-Acetyl-β-D-Glusoaminidase) bestimmt. Auf der anderen Seite konnte in vergleichbaren Makrophagenkulturen die Pinozytose durch die Aufname von kolloidalem Gold ($^{198}Au$)—wie sie dem Fachmann bekannt ist—untersucht werden. Die Höhe des oxidativen Stoffwechsels bei Makrophagen gilt als ein weiteres Maß für ihren Aktivierungszustand. Gemessen wird diese Aktivität unter Zuhilfenahme des Biolumaten durch Bestimmung der Chemolumineszenz.

Zu diesem Zweck wurden entweder in Petrischalen von 30 mm Durchmesser $3\times10^6$ Makrophagen mit 1 ml TC 199-Kulturmedium oder aber $10^6$ Makrophagen mit 100 µl in Rundbogen-Polyäthylen-Röhrchen (zur Bestimmung der Chemolumineszenz) bei 5% $CO_2$ und 37°C kultiviert.

Nach einstündiger Bebrütung werden die Kulturen gewaschen, um schwimmende Zellen zu entfernen. Die Chemolumineszenz (Röhrchenkultur) wurde dann direkt bestimmt, während die Petrischalen erneut 24 Stunden bei 37°C bebrütet wurden und danach die Enzym- und Pinozytoseaktivität in den Kulturen bestimmt wurde. Es wurden die nachstehenden Ergebnisse erzielt.

TABELLE 2
Wirkung auf den oxidativen Stoffwechsel bei Maus-Peritonealmakrophagen (Chemolumineszenz in RLE*/15 Minuten)

| 1×Applikation von | | intraperitoneal | oral |
|---|---|---|---|
| PBS | | 2,97∓0,28×$10^5$ | 3,65∓0,81×$10^5$ |
| Prüfsubstanz | 25 mg/kg | 7,87∓0,28×$10^5$ | 6,41∓0,42×$10^5$ |
| | 50 mg/kg | 9,72∓0,82×$10^5$ | 8,99∓0,39×$10^5$ |
| | 100 mg/kg | 12,85∓2,37×$10^5$ | 11,85∓0,92×$10^5$ |
| | 200 mg/kg | 24,40∓3,39×$10^5$ | 15,60∓1,98×$10^5$ |

*RLE=Relative Lichteinheiten.

Die parenterale sowie die orale Behandlung von NMRI-Mäusen mit der gemäß Beispiel 8 hergestellten Prüfsubstanz stimuliert die Makrophagenaktivität und hat damit eine immunitätstimulierende Wirkung. So wird der oxidative Stoffwechsel bei Makrophagen mit der Generierung von Sauerstoffradikalen und dem damit verbundenen meßbaren Licht deutlich erhöht. Bei Dosierungen von 25 mg/kg aufwärts kommt es zu einer dosisabhängigen Steigerung der Makrophagenaktivität sowohl bei parenteraler als auch oraler Applikation.

Aus der Tabelle 3 ist zu entnehmen, daß Makrophagen von Kontrollmäusen nur geringe Mengen an lysosomalen Enzymen (β-Glucuronidase, β-Galactosidase, N-Acetyl-β-D-Glucosaminidase) in den Kulturüberstand abgeben. Mononukleäre Phagozyten von Mäusen, die parenteral oder oral mit der Prüfsubstanz 72 Stunden behandelt wurde, sezernieren die o.a. sauren Hydrolasen (β-Glu, β-Gal, N-Ac-Glu) deutlich mehr und weisen damit eine Dosiswirkungskurve auf, die bei allen gemessenen Enzymen eine Überlegenheit gegenüber den Kontrollen erkennen läßt. Es ist ersichtlich, daß die Prüfsubstanz eine stimulierende Wirkung auf die Makrophagenaktivität besitzt und zur Erhöhung der Enzymfreisetzung beiträgt.

TABELLE 3
Einfluß der Prüfsubstanz auf die Enzymfreisetzung lysosomaler Hydrolasen von Maus-Peritonealmakrophagen

| 1×i.p./p.o. Applikation | | β-Glu mU/ml | β-Gal mU/ml | N-Ac-Glu mU/ml |
|---|---|---|---|---|
| PBS | | 755/484 | 1306/1702 | 1238/1168 |
| Prüfsubstanz | 25 mg/kg | 1001/897 | 2584/4917 | 2786/1947 |
| | 50 mg/kg | 1370/1133 | 11058/ 9179 | 3315/2862 |
| | 100 mg/kg | 1791/1593 | 17597/13195 | 4305/3676 |
| | 200 mg/kg | 2136/1886 | 22351/17357 | 6548/5621 |

Die quantitative Bestimmung der Pinozytoseaktivität bei mononukleären Phagozyten wurde nach der Methode von Davies et al. durchgeführt (Davies, P. Allison, A. C. und Haswell, A. D.; Biochem. Biophys. Res. Com. *52*, 627, 1973). Dazu wurde radioaktives, kolloidales Gold ($^{198}$Au) mit einer Partikelgröße von 20 nm und einer spezifischen Aktivität von 4—12 mCi/mg Au benutzt. Die Ergebnisse in Tabelle 4 veranschaulichen den Effekt der nach Beispiel 8 erhaltenen Prüfsubstanz auf die Endozytoseleistung. Die Pinozytose von kolloidalem Gold ($^{198}$Au) durch Maus-Peritonealmakrophagen von mit der erfindungsgemäßen Verbindung behandelten Tieren ist im Vergleich mit den Makrophagen von unbehandelten Tieren signifikant und dosisabhängig erhöht.

TABELLE 4
Der Effekt der Prüfsubstanz auf die Pinozytoseleistung von Mausmakrophagen

| 1×Applikation von | | intraperitoneal | oral |
|---|---|---|---|
| PBS | | 0,286×$10^3$ cpm | 0,198×$10^3$ cpm |
| Prüfsubstanz | 25 mg/kg | 0,341×$10^3$ cpm | 0,272×$10^3$ cpm |
| | 50 mg/kg | 0,396×$10^3$ cpm | 0,358×$10^3$ cpm |
| | 100 mg/kg | 0,462×$10^3$ cpm | 0,416×$10^3$ cpm |
| | 200 mg/kg | 0,587×$10^3$ cpm | 0,506×$10^3$ cpm |

Experiment 3

Erhöhung der Widerstandskraft von Balb/c-Mäusen gegen eine Candida albicans Infektion

a) Prophylaktische Behandlung:

Balb/c-Mäuse wurden über 4 Tage in einer Dosierung von 2×60 mg/kg/Tag intraperitoneal mit der Prüfsubstanz (Verbindung nach Ausführungsbeispiel 8) behandelt. 24 Stunden nach der letzten Gabe der Prüfsubstanz werden diese Tiere und die Kontrolltiere, denen physiologische Kochsalzlösung in gleichen Volumina und Zeitabständen verabreicht worden war, mit Candida albicans intravenös infiziert ($5\times10^5$ CFU/Maus). Aus der Absterberate nach der Infektion lassen sich entsprechend die mittleren Überlebenszeiten berechnen. Die Tiere der Kontrollgruppe sterben zu 50% nach 9,7 Tagen, die mit der Prüfsubstanz behandelte Gruppe zeigte eine mittlere Überlebenszeit von 16,1 Tagen. In dem gewählten Applikationsschema mit den entsprechenden Dosierungen (prophylaktische Verabreichung) induziert die Prüfsubstanz eine signifikante Erhöhung der Resistenz der Balb/c-Maus gegen Candida albicans.

TABELLE 5

Mittlere Überlebenszeiten nach C. albicans Infektion ($5\times10^5$ CFU)

| Substanz 2×60 mg/kg/ Tag i.p. | mittlere Überlebens-zeit (Tage) | Vertrauensbereich 95% | Vertrauensbereich 99% |
|---|---|---|---|
| PBS | 9,7 | 8,4—10,6 | 7,8—10,9 |
| Prüfsubstanz | 16,1 | 14,8—17,4 | 14,4—17,8 |

b) Therapeutische Behandlung:

Bei der therapeutischen Behandlung einer chronischen Candida albicans Infektion wurden weibliche Balb/c-Mäuse (15/Gruppe) am Tag 0 intravenös mit Candida albicans ($1\times10^5$ CFU/Maus) infiziert. Nach erfolgter Infektion wurden die Tiere an 8 aufeinanderfolgenden Tagen (Tag 3—10) mit jeweils 60 mg/kg der Prüfsubstanz (Verbindung nach Ausführungsbeispiel 8) intraperitoneal behandelt. Den Kontrolltieren wurde physiologische Kochsalzlösung injiziert. An den Tagen 8, 14 und 21 wurden den Tieren Urin entnommen und die Keimzahl bestimmt. Am Tag 30 wurden die Tiere getötet und die Keimzahl und die Nekrosenbildung der Nieren bestimmt. Die *Tabelle 6* zeigt, daß die Prüfsubstanz bei therapeutischer Gabe deutliche alle Parameter der chronischen Candida albicans Infektion (Keimzahl in Urin und Nieren und Nekrosenbildung) reduziert und damit die Erkrankung therapeutisch beeinflußt. Während bei den Kontrolltieren sich zu einem hohen Prozentsatz Keime im Urin nachweisen lassen, wird bei den behandelten Tieren die Keimzahl signifikant erniedrigt.

Auch die Keimbesiedlung der Nieren wird durch die Therapie von 63% auf 27% reduziert, bei einer gleichzeitigen Verringerung der Nekrosenbildung von 87% auf 10%.

TABELLE 6

Therapeutische Behandlung einer chronischen Candida albicans Infektion ($1\times10^5$ CFU)

| Substanz 60 mg/kg i.p. Tag 3—10 | Tiere mit positivem Befund Tag 8 | Tiere mit positivem Befund Tag 14 | Tiere mit positivem Befund Tag 21 | Nieren mit positivem Keimbefund Tag 30 | nekrotische Nieren Tag 30 |
|---|---|---|---|---|---|
| PBS | 4/15 (27%) | 8/15 (53%) | 10/15 (67%) | 19/30 (63%) | 26/30 (87%) |
| Prüfsubstanz | 1/15 (7%) | 1/15 (7%) | 4/15 (27%) | 8/30 (27%) | 3/30 (10%) |

Experiment 4

Stimulation der DTH-Reaktion durch die erfindungsgemäßen Verbindungen

Wie in Experiment 1 beschrieben, wurden NMRI-Mäuse mit den erfindungsgemäßen Substanzen behandelt.

Als Test zur Erfassung der Immunstimulation wurde die DTH-Reaktion überprüft.

*Tabelle 7* zeigt die relative Wirksamkeit der Prüfsubstanz, bezogen auf die Verbindung aus Beispiel 8, deren maximale Aktivierung 100% entspricht (Differenz zwischen Kontrolle und Stimulation). Der Tabelle ist zu entnehmen, daß die DTH-Reaktion bei den mit den Prüfsubstanzen vorbehandelten Tieren deutlich stärker ausgeprägt ist, als bei den entsprechenden Kontrolltieren.

TABELLE 7

| Verbindung aus Beispiel Nr. | Dosis (mg/kg) | Applikation | DTH-Reaktion (SRBC) |
|---|---|---|---|
| 2 | 20 | 1×i.p. Tag 0 | 88% |
| 5 | 200 | " | 61% |
| 6 | 100 | " | 100% |
| 8 | 100 | " | 100% |
| 10 | 10 | " | 112% |
| 12 | 100 | " | 96% |
| 13 | 200 | " | 147% |
| 14 | 100 | " | 118% |

Experiment 5
Stimulation der Makrophagenaktivität durch die erfindungsgemäßen Verbindungen:

Wie im Experiment 2 beschrieben, wurden NMRI-Mäuse mit den erfindungsgemäßen Verbindungen behandelt.

Als Test zur Erfassung der Immunstimulation wurde die Funktion der Makrophagen (Chemolumineszenz und Enzymaktivität) überprüft.

*Tabelle 8* zeigt die relative Wirksamkeit der Prüfsubstanzen bezogen auf die Verbindung aus Beispiel 8, deren maximale Aktivierung (Differenz zwischen Kontrolle und Stimulation) 100% entspricht. Der Tabelle ist zu entnehmen, daß im Vergleich zu Makrophagen aus unbehandelten Tieren, diese Zellen auch durch alle Prüfsubstanzen in ihrer Chemolumineszenzreaktion stark stimuliert und in ihrem Gehalt an lysosomalen Enzymen deutlich erhöht waren.

TABELLE 8

| Verbindung aus Beispiel Nr. (100 mg/kg i.p.) | Makrophagenaktivität | |
|---|---|---|
| | Chemolumineszenz | Exozytose |
| 1 | 30% | 48% |
| 2 | 72% | 53% |
| 5 | 37% | 45% |
| 6 | 26% | 33% |
| 8 | 100% | 100% |
| 10 | 66% | 21% |
| 12 | 39% | 54% |
| 13 | 81% | 77% |
| 14 | 97% | 93% |

Experiment 6
Stimulation der Infektabwehr gegen Candida albicans Infektionen durch prophylaktische Gabe der erfindungsgemäßen Verbindungen

Wie in Experiment 3a beschrieben, wurden Balb/c-Mäuse mit den erfindungsgemäßen Verbindungen behandelt.

Wie *Tabelle 9* zeigt, steigt die mittlere Überlebenszeit der Mäuse nach C. albicans Infektion, verglichen mit einer unbehandelten Kontrollgruppe, signifikant an.

TABELLE 9

| Beispiel Nr. | Dosis (mg/kg) 1×i.p. Tag | Relative Verlängerung der mittleren Überlebenszeiten* post inf. |
|---|---|---|
| 1 | 1 | 144 |
| 2 | 1 | 123 |
| 5 | 10 | 142 |
| 6 | 10 | 145 |
| 8 | 1 | 130 |
| 10 | 10 | 135 |
| 12 | 10 | 205 |
| 14 | 10 | 107 |

*Mittlere Überlebenszeit der Kontrollgruppe=100.

Experiment 7

Einfluß auf die Stimulation der Tumorabwehr gegen das B16-Melanom

Bei weiblichen C57B1/6 Mäusen (10 Tiere/Gruppe) mit einem Gewicht von 18—20 g wurde mit $2\times10^5$ lebenden B16-Melanomzellen ein Primärtumorwachstum induziert. Nach Ausprägung einer bestimmten Tumorgröße (0.65 cm im Durchmesser) wurde der Primärtumor entfernt. Die unbehandelten Tiere starben dann an Metastasen in der Lunge. Nach der Tumorinduktion wurden die Tiere an den Tagen 3, 5, 7, 9, 11, und 13 nach Amputation des Primärtumors mit 50, 100 und 200 mg/kg der nach Beispiel 8 erhaltenen Prüfsubstanz intraperitoneal behandelt. Aus der Absterberate nach Amputation des Primärtumors durch die Entwicklung von Lungenmetastasen lassen sich entsprechend die mittleren Überlebenszeiten berechnen. Danach sterben die Tiere der Kontrollgruppe zu 50% nach 26 Tagen. Die mit der Prüfsubstanz behandelten Gruppen zeigten (vgl. *Tabelle 10*) mit den entsprechenden Dosierungen eine signifikante Erhöhung der mittleren Überlebenszeit auf 43, 40 und 35 Tage.

TABELLE 10

Therapeutische Tumor-Behandlung beim B16-Melanom

| Applikation 6×i.p. (mg/kg) Tag 3, 5, 7, 9, 11, 13 | mittlere Überlebenszeit (Tage) |
|---|---|
| PBS | 26 |
| Prüfsubstanz 50 | 43 |
| Prüfsubstanz 100 | 40 |
| Prüfsubstanz 200 | 35 |

Experiment 8

Einfluß auf die Knochenmarkskoloniebildung

Hier wurde der Einfluß der nach Beispiel 8 erhaltenen Prüfsubstanz auf die Stimulation von Knochenmarkskolonien bei 6—8 Wochen alten B2D2F1 Mäusen untersucht. Mäuseweibchen erhielten die Prüfsubstanz intraperitoneal in den Dosen 2,5 und 5 mg/kg. Einen Tag später wurden die Tiere getötet, die Knochenmarkszellen isoliert und nach den allgemein bekannten Methoden (Metcalf, Immunology *21*, 427, 1971 und Stanley et al. J. Exp. Med. *143*, 631, 1979) kultiviert. Zur Entwicklung der Knochenmarkskolonien wurde als "CSF"-Quelle (Colony stimulating factor) wie üblich L-Zell-Überstand (15%) benutzt. Die Kolonien wurden 8 Tage nach der Aussaat gezählt. Wie aus der *Tabelle 11* zu ersehen ist, führt die

einmalige Gabe von 2,5 oder 5 mg der Prüfsubstanz zu einer deutlichen Steigerung der Koloniebildung bei Knochenmarkszellen sowohl mit als auch ohne Zugabe von CSF (Colony stimulating factor) in vitro.

TABELLE 11
In vivo-Effekt auf die Knochenmarkskoloniebildung

| Prüfsubstanz 1×i.p. (mg/kg) | Zahl der Knochenmarkskolonien (Tag 8) | |
|---|---|---|
| | mit CSF (15%) | ohne CSF (15%) |
| PBS | 41∓6 | 0 |
| Prüfsubstanz 2,5 | 94∓11 | 24∓2 |
| 5,0 | 163∓8 | 44∓5 |

Die folgenden Beispiele erläutern die Erfindung.

Beispiel 1
Bis-(3-Hydroxy-1-phenyl-1,2,4-triazol-5-yl)-disulfid
2,9 g (15 mmol) 3-Hydroxy-1-phenyl-5-mercapto-1,2,4-triazol werden in, 100 ml Methanol vorgelegt und bei Raumtemperatur 3,2 ml (33 mmol) Wasserstoffperoxid 35 %ig in Methanol zugetropft. Die Lösung färbt sich zunächst gelb und nach 20 Min. fällt die Verbindung langsam aus. Nach vier Stunden wird der Niederschlag abgesaugt, mit Methanol gewaschen und getrocknet.
Ausbeute 2,1 g=72% d. Th.
Fp: 232°C, Zersetzung.
NMR ($d_6$-DMSO) δ=11,58 ppm, s, 2H, OH
7,38 ppm, m, 10H, aromat. H.

Beispiel 2
Bis-(5-Carboxy-benzimidazol-2-yl)-disulfid
Darstellung analog Beispiel 1 aus 2-Mercaptobenzimidazol-5-carbonsäure.
Ausbeute 70% d. Th.
Fp. 235—38°C.
NMR ($d_6$-DMSO) δ=9,4 ppm, d, NH
7,4—8,4 ppm, m, aromat. H.

Beispiel 3
Bis-[4-(2-Chlorallyl)-6-hydroxy-5-oxo-4,5-dihydro-1,2,4-triazin-3-yl]-disulfid
2,2 g 4-(2-Chlorallyl)-6-hydroxy-3-mercapto-5-oxo-4,5-dihydro-1,2,4-triazin wurden in 25 ml Methanol gelöst, bei Raumtemperatur tropfenweise mit 0,86 ml 35% $H_2O_2$ versetzt und 1 Stunde nachgerührt wobei das Produkt auskristallisierte. Es wurde abgesaugt, mit Methanol gewaschen und an der Luft getrocknet.
Ausbeute 1,2 g
Fp: 204—05°C, (Z)
NMR ($d_6$-DMSO) δ=12,7 ppm (br. s., OH)
5,5 ppm (dd, $=CH_2$)
4,8 ppm (s, $CH_2N$).

Beispiel 4
Bis-(6-Hydroxy-2-methyl-5-oxo-2,5-dihydro-1,2,4-triazin-3-yl)-disulfid
1,59 g 6-hydroxy-3-mercapto-2-methyl-5-oxo-2,5-dihydro-1,2,4-triazin wurden in 30 ml Methanol gelöst, bei Raumtemperatur tropfenweise mit 0,43 ml 35% $H_2O_2$ versetzt und 30 Minuten nachgerührt. Die Lösung wurde mit Kohle geklärt, einrotiert und der Rückstand mit Eiswasser verrieben. Der Niederschlag wurde abfiltriert, mit Eiswasser gewaschen, an der Luft getrocknet, in Methanol suspendiert, abfiltriert, mit wenig Methanol gewaschen und im Vakuum getrocknet.
Ausbeute 0,8 g
Fp: 220°C (Z)
NMR ($d_6$-DMSO) δ=3,87 ppm (s, $CH_3$).

Beispiel 5
Bis-(6-Hydroxy-4-methyl-5-oxo-4,5-dihydro-1,2,4-triazin-3-yl)-disulfid
3,7 g 6-Hydroxy-3-mercapto-4-methyl-5-oxo-4,5-dihydro-1,2,4-triazin wurden in 400 ml Methanol und

38 ml $H_2O$ gelöst. Bei Raumtemperatur wurden

2,5 ml 35% $H_2O_2$ zugetropft. Nach 30 Minuten Rühren wurde auf 100 ml eingeengt, der Niederschlag abgesaugt und getrocknet.

Ausbeute 1,5 g

Fp: 237°C

NMR ($CF_3CO_2D$): δ=3,67 ppm s, 2H, OH

3,8 ppm, s, 6H, $CH_3$.

Beispiel 6

Bis-(5-Carboxymethyl-4-methyl-1,3-thiazol-2-yl)-disulfid

23,62 g 5-Carboxymethyl-4-methyl-2-mercapto-1,3-thiazol wurden in

250 ml Methanol gelöst, filtriert und unter Wasserbadkühlung langsam mit

12,5 ml 35% $H_2O_2$ versetzt. Es wurden 30 Minuten im Wasserband und 2 Stunden bei Raumtemperatur nachgerührt. Nach Filtration und Waschen mit Methanol wurden 22,8 g der Titelverbindung isoliert.

FP: 162°C

NMR ($CF_3CO_2D$): δ=2,6 ppm, s, 6H, $CH_3$

4,2 ppm, s, 4H, $CH_2$.

Beispiel 7

Bis-(5-Carboxymethyl-1,3-thiazol-2-yl)-disulfid

0,88 g 2-Mercapto-1,3-thiazol-5-yl-essigsäure werden in ca. 10 ml Methanol gelöst und unter Rühren bei Raumtemperatur mit

0,5 ml 33% $H_2O_2$-Lösung versetzt. Nach 0,5 h wurde die Kristallsuspension gekühlt, filtriert und der Filterrückstand getrocknet. Ausbeute 0,6 g vom Zersetzungspunkt 150°C. Dünnschichtchromatographischer Vergleich auf Merck-Silicagel-Platten zeigt vollständige Umsetzung (RF: 0,3; Laufmittel: Essigester: 65, Ethanol: 25, Wasser: 10, Ameisensäure: 1).

Beispiel 8

Bis-(4-Carboxymetyyl-1,3-thiazol-2-yl)-disulfid

Setz man

1,75 g 4-Carboxymethyl-2-mercapto-1,3-thiazol analog Beispiel 7 um, so erhält man 0,95 g der Titelverbindung.

Fp: 163—65°C

NMR (DMSO-$d_6$): δ=3,7 ppm, s, 4H, $CH_2$

7,6 ppm, s, 2H, CH

9,4 ppm, s, 2H, $CO_2H$.

Beispiel 9

Bis-(2-Carboxymethylthio-1,3,4-thiadiazol-5-yl)-disulfid

1,4 g (2-Mercapto-1,3,4-thiadiazol-5-yl)-mercaptoessigsäure werden in 10 ml Methanol aufgelöst und unter Eiskühlung mit

0,65 ml 35% $H_2O_2$ tropfenweise versetzt. Es wird 1 Stunde bei Raumtemperatur nachgerührt und filtriert. Die Kristalle werden mit wenig Methanol nachgewaschen und im Vakuum getroknet.

Ausbeute: 1,3 g

Fp: 179°C, (Z)

NMR (DMSO-$d_6$): δ=13 ppm, breit, $CO_2$

4,2 ppm, s, $CH_2$.

Beispiel 10

Bis-(5-Carboxy-1,3-thiazol-2-yl)-disulfid

21 g 2-Mercapto-1,3-thiazol-5-carbonsäure werden in

100 ml Methanol unter leichtem Erwärmen gelöst. Unter Eiskühlung wird langsam mit.

10,7 ml 35% $H_2O_2$ versetzt und 45 Minuten bei Raumtemperatur nachgerührt. Es wird abgesaugt, mit Methanol gewaschen und im Vakuum getrocknet.

Ausbeute 21,5 g

Fp: 280°C, (Z)

NMR (DMSO-$d_6$): δ=8,48 ppm, s, Thiazol-H.

Beispiel 11

Bis-(5-Carboxy-4-methyl-1,3-thiazol-2-yl)-disulfid

1,75 g (10 mmol) 4-Methyl-2-mercapto-1,3-thiazol-5-carbonsäure werden in

50 ml Methanol aufgeschlämmt und unter Eiskühlung mit

0,86 ml 35% $H_2O_2$ versetzt. Die Lösung wird 1/2 Stunde bei 0°C und 1 Stunde bei Raumtemperatur

nachgerührt. Die gebildeten Kristalle werden abgesaugt mit wenig Methanol gewaschen und im Vakuum getrocknet.

Ausbeute 1,52 g; Fp: 240—41°C, (Z)

NMR (DMSO-$d_6$): δ=2,6 ppm, s, $CH_3$.

Beispiel 12

Bis-(4-Carboxymethyl-5-methyl-1,3-thiazol-2-yl)-disulfid

Stufe 1

4-Brompropionylessigsäuremethylester

22 g Propionylessigsäuremethylester werden in 60 g $CH_2Cl_2$ gelöst und tropfenweise mit 8,7 ml $Br_2$ in 30 ml $CH_2Cl_2$ versetzt. Es wird 1-1/4 h bei Raumtemperatur nachgerührt, nochmals mit 0,66 ml $Br_2$ in 5 ml $CH_2Cl_2$ versetzt und weitere 1-1/4 h gerührt. Es wird 3×mit je 50 ml $H_2O$, 1×mit 20 ml ges. $NaHCO_3$ und 2×mit je 50 ml $H_2O$ gewaschen, über $Na_2SO_4$ getrocknet und eingeengt. Man erhält 38,6 g Öl.

Stufe 2

(2-Mercapto-5-methyl-1,3-thiazol-4-yl)-essigsäuremethylester

30,1 g des Öls aus Stufe 1, gelöst in 80 ml Äthanol, werden zu einer Lösung von 24 g frisch bereitetem Ammoniumdithiocarbaminat in 200 ml Äthanol/200 ml $H_2O$ beim Raumtemperatur zugetropft. Es wird 2-1/2 h nachgerührt und mit 7,5 ml Trifluoressigsäure versetzt. Nach 1 Stunde Nachrühren wird zur Trockne eingedampft und mit 200 ml $CHCl_3/H_2O$ (1:1) versetzt. Es wird noch zweimal mit $H_2O$ gewaschen, über $Na_2SO_4$ getrocknet und eingeengt. Der fest Rückstand wird in Äther aufgeschlämmt und abfiltriert.

Ausbeute 8,9 g

Fp: 149°C, (Z)

NMR ($d_6$-DMSO): δ=11 ppm, breit, SH
3,7 ppm, s, $OCH_3$
3,5 ppm, s, $CH_2$
2,1 ppm, s, $CH_3$.

Stufe 3

(2-Mercapto-5-methyl-1,3-thiazol-4-yl)-essigsäure

1,98 g aus Stufe 2 wurden unter Erwärmen in 20 ml Methanol gelöst. Es wurde 40 Min. bei Raumtemperatur nachgerührt, mit 50 ml $H_2O$ versetzt, und mit HCl konz. auf pH 1,0 angesäuert. Nach 1 h Rühren unter Eiskühlung wurde abgesaugt und mit Eiswasser chloridfrei gewaschen. Trocknen im Vakuum über $P_2O_5$ ergaben 1,67 g.

NMR ($d_6$-DMSO): δ=13,9 ppm, breit, $CO_2H$
3,5 ppm, s, $CH_2$
2,08 ppm, s, $CH_3$.

Stufe 4

Bis-(4-Carboxymethyl-5-methyl-1,3-thiazol-2-yl)-disulfid

430 mg aus Stufe 3 wurden in 20 ml Methanol suspendiert und unter Erwärmen gelöst. Bei Raumtemperatur wurden 0,25 ml 35% $H_2O_2$ zugetropft und noch 1 h nachgerührt. Nach Einengen auf ca. 5 ml wurde filtriert, und mit wenig ausgekühltem Methanol gewaschen.

Ausbeute: 280 mg

NMR ($d_6$-DMSO): δ=3,65 ppm, s, $CH_2$
2,37 ppm, s, $CH_3$.

Beispiel 13

Bis-(5-Carboxymethyl-1,3-thiazol-2-yl)-disulfid

0,8 g 2-Mercapto-5-carboxymethyl-1,3-thiazol wurden in 10 ml Methanol gelöst und unter Rühren tropfenweise mit 0,5 ml 33 %iger $H_2O_2$-Lösung versetzt. Man ließ über Nacht rühren, und filtrierte das auskristallisierte Produkt ab.

Ausbeute 0,6 g vom Fp. 150°C (Zers.), dünnschichtchromatographisch einheitlich (Rf=0,3; Kieselgel; Laufmittel: Essigester, Ethanol, Wasser, Ameisensäure (60:25:15:1).

IR (KBr-Preßling): v=1710 cm (COOH).

$^1$H-NMR ($d_6$-DMSO): δ(ppm): 3,8 (s, $CH_2$-Thiazol, 2H),
7,6 (s, Thiazol-4H, 1H).

Beispiel 14

Bis-(4-Carboxy-1,3-thiazol-5-yl)-disulfid

5 g Bis-(4-methoxycarbonyl-1,3-thiazol-5-yl)-disulfid wurden in 100 ml Methanol suspendiert. Dazu gab man eine Lösung von 1,5 g NaOH in 20 ml $H_2O$ und erhitzte 1 h unter Rückfluß. Nach dem Abkühlen der Lösung wurde Methanol am Rotationsverdampfer abgezogen, die Wasserphase einmal mit wenig Essigester extrahiert und mit 2 N HCl sauer gestellt. Das Produkt wurde abfiltriert, getrocknet und aus Essigester umkristallisiert. Man erhielt 2 g vom Fp. 154°C.

IR (KBr-Preßling): $\nu=1680\ cm^{-1}$ (COOH)

$^1H$-NMR ($d_6$-DMSO): δ (ppm), 8,6 (s, Thiazol-2H).

Die Biespiele 15 bis 17 wurden wie in Beispiel 1 beschrieben durchgeführt.

Beispiel 15

Bis-(5-Glutarsäuremonoamido-1,3-thiazol-2-yl)-disulfid

1H-NMR ($d_6$-DMSO):

δ=1,6—2,6 ppm (m, 12H, sechs $CH_2$-Gruppen), 7,50 ppm (s, 2H, Thiazol-H), 12,63 ppm (br s, 2H, —NH—), 13,05 ppm (br s, 2H, —$CO_2H$).

Beispiel 16

Bis-(5-Bernsteinsäuremonoamido-1,3-thiazol-2-yl)-disulfid

1H-NMR ($d_6$-DMSO):

δ=2,4—2,7 ppm (m, 8H, —$CH_2$), 7,50 ppm (s, 2H, Thiazol-H), 12,66 ppm (br s, 2H, —NH—), 13,15 ppm (br s, 2H, —$CO_2H$).

Beispiel 17

Bis-[5-(2-Carboxyethyl-1-yl)-4-methyl-1,3-thiazol-2-yl]-disulfid

1H-NMR ($d_6$-DMSO):

δ=2,27 ppm (s, 6H, —$CH_3$), 2,55 ppm (d, 4H, Thiazol-$CH_2$), 2,96 ppm (t, 4H, $CH_2$—$CO_2$—).

**Patentansprüche**

1. Disulfide der allgemeinen Formel I'

$$\text{Het'—S—S—Het'} \quad (I')$$

in der Het' die Bedeutung besitzt von 1,3-Thiazolyl, das substituiert ist durch $C_1$—$C_6$-Alkyl; durch Carboxy-$C_1$—$C_6$-alkyl; durch Carboxy oder durch $C_2$—$C_5$-Acylamino, wobei Acyl der Rest einer aliphatischen Dicarbonsäure ist; 1,2,4-Triazolyl, das substituiert ist durch Hydroxy und Phenyl; 1,3,4-Thiadiazolyl, das substituiert ist durch Carboxy-$C_1$—$C_4$-alkylthio; Dihydro-1,2,4-triazinyl, das substituiert ist durch $C_1$—$C_6$-Alkyl, Halogeno-$C_2$—$C_4$-alkenyl, Oxo oder Hydroxy oder Benzimidazolyl, das substituiert ist durch Carboxy, wobei Het' durch mindestens eine direkt oder an einen Substituenten gebundene Carboxygruppe oder eine direkt gebundene Hydroxygruppe, wobei diese beiden Gruppen auch in Form eines physiologisch verträglichen Salzes vorliegen können, substituiert ist.

2. Disulfide der allgemeinen Formel I'

$$\text{Het'—S—S—Het'} \quad (I')$$

in der Het' die Bedeutung besitzt von 1,3-Thiazolyl, das ein- oder zweifach substituiert ist durch $C_1$—$C_3$-Alkyl; durch Carboxy-$C_1$—$C_3$-alkyl; durch Carboxy oder durch $C_2$—$C_5$-Acylamino, wobei Acyl der Rest einer aliphatischen Dicarbonsäure ist, wobei Het' durch mindestens eine direkt oder an einen Substituenten gebundene Carboxygruppe, die auch in Form eines physiologisch verträglichen Salzes vorliegen kann, substituiert ist.

3. Verfahren zur Herstellung der Disulfide der allgemeinen Formel I', dadurch gekennzeichnet, daß man

a) eine Verbindungen der allgemeinen Formel II

$$\text{Het'—SH} \quad (II)$$

in der Het' die vorstehende Bedeutung hat, durch Umsetzung mit einem Oxidationsmittel in eine Verbindung der allgemeinen Formel I' überführt oder

b) eine Verbindung der allgemeinen Formel III

$$\text{Het'—X} \quad (III)$$

in der Het' die vorstehende Bedeutung hat und X für eine rekative, leicht abspaltbare Gruppe steht, mit $Me_2S_2$ umsetzt, wobei Me für ein Alkalimetall steht, oder

c) eine Verbindung der allgemeinen Formel IV

$$Het'—S—SO_2Me \quad (IV)$$

in der Het und Me die vorstehenden Bedeutungen haben, mit Jod in wäßrigem Medium unsetzt.

4. Pharmazeutische Mittel gekennzeichnet durch einen Gehalt an einer Verbindung der allgemeinen Formel I'.

**Revendications**

1. Disulfures de formule générale I'

$$Het'—S—S—Het' \quad (I')$$

dans laquelle Het' a la signification d'un radical 1,3-thiazolyle, qui est substitué par un groupe alkyle en $C_1$—$C_6$, par un groupe carboxy-alkyle($C_1$—$C_6$), par le groupe carboxy ou par un groupe acylamino en $C_2$—$C_5$, le fragment acyle étant le reste d'un acide dicarboxylique aliphatique; d'un radical 1,2,4-thiazolyle, qui est substitué par le groupe hydroxy et le groupe phényle; d'un radical 1,3,4-thiadiazolyle, qui est substitué par un groupe carboxy-alkyl($C_1$—$C_4$)-thio; d'un radical dihydro-1,2,4-triazinyle, qui est substitué par un groupe alkyle en $C_1$—$C_6$, halogéno-alcényle($C_2$—$C_4$), oxo ou hydroxy; ou d'un radical benzimidazolyle, qui est substitué par le groupe carboxy, Het' étant substitué par au moins un groupe carboxy lié directement ou à un substituant, ou un groupe carboxy lié directement, ces deux groupes pouvant également être présents sous forme d'un sel physiologiquement acceptable.

2. Disulfures de formule générale I'

$$Het'—S—S—Het' \quad (I')$$

dans laquelle Het' représente un radical 1,3-thiazolyle qui est substitué une ou deux fois par un groupe alkyle en $C_1$—$C_3$, par un groupe carboxy-alkyle en $C_1$—$C_3$, par un groupe carboxy ou par un groupe acylamino en $C_2$—$C_5$, le fragment acyle étant le reste d'un acide dicarboxylique aliphatique, Het' étant substitué par au moins un groupe carboxy lié directement ou à un substituant, groupe carboxy qui peut également se trouver sous forme d'un sel physiologiquement acceptable.

3. Procédé pour la préparation desdits sulfures de formule générale I', caractérisé en ce que

a) on convertit en un composé de formule générale I' un composé de formule générale II

$$Het'—SH \quad (II)$$

dans laquelle Het' la signification donnée plus haut, par réaction avec un oxydant, ou

b) on fait réagir un composé de formule générale III

$$Het'—X \quad (III)$$

dans laquelle Het' a la signification donnée plus haut et X représente un groupe réactif, aisément séparable, avec $Me_2S_2$, Me représentant un métal alcalin, ou

c) on fait réagir un composé de formule générale IV

$$Het'—S—SO_2Me \quad (IV)$$

dans laquelle Het' et Me ont les significations données plus haut, avec de l'iode dans un milieu aqueux.

4. Compositions pharmaceutiques caractérisées par une teneur en un composé de formule générale I'.

**Claims**

1. A disulfide of the formula I'

$$Het'—S—S—Het' \quad (I')$$

in which Het' means 1,3-thiazolyl which is substituted by $C_1$—$C_6$-alkyl; by carboxy-$C_1$—$C_6$-alkyl; by carboxyl or by $C_2$—$C_5$-acylamino, where acyl is the radical of an aliphatic dicarboxylic acid; 1,2,4-triazolyl, which is substituted by hydroxyl and phenyl; 1,3,4-thiadiazolyl, which is substituted by carboxy-$C_1$—$C_4$-alkylthio; dihydro-1,2,4-triazinyl which is substituted by $C_1$—$C_6$-alkyl, halogeno-$C_2$—$C_4$-alkenyl, oxo or hydroxyl or benzimidazolyl which is substituted by carboxyl, where Het' is substituted by at least one carboxyl group which is bonded directly or to a substituent or by one directly bonded hydroxyl group, it also being possible for these two groups to be in the form of a physiologically tolerated salt.

2. A disulfide of the formula I'

$$Het'—S—S—Het' \quad (I')$$

in which Het' means 1,3-thiazolyl which is substituted once or twice by $C_1$—$C_3$-alkyl; by carboxy-$C_1$—$C_3$-alkyl; by carboxyl or by $C_2$—$C_5$-acylamino, where acyl is the radical of an aliphatic dicarboxylic acid, where Het' is substituted by at least one carboxyl group which is bonded directly or to a substituent and can also be in the form of a physiologically tolerated salt.

3. A process for the preparation of disulfides of the formula I', which comprises

a) converting a compound of the formula II

$$Het'—SH \quad (II)$$

in which Het' has the abovementioned meaning, by reaction with an oxidizing agent into a compound of the formula I', or

b) reacting a compound of the formula III

$$Het'—X \quad (III)$$

in which Het' has the abovementioned meaning, and X is a reactive group which can easily be eliminated, with $Me_2S_2$, where Me is an alkali metal, or

c) reacting a compound of the formula IV

$$Het'—S—SO_2Me \quad (IV)$$

in which Het' and Me have the abovementioned meanings, with iodine in aqueous medium.

4. A pharmaceutical agent which contains a compound of the formula I'.